(19) Europäisches Patentamt

European Patent Office

Office européen des brevets

(11) **EP 0 917 476 B1**

(12) **EUROPEAN PATENT SPECIFICATION**

(45) Date of publication and mention
of the grant of the patent:
**29.09.2004 Bulletin 2004/40**

(51) Int Cl.$^7$: **A61M 15/00**, A61M 16/00,
B05B 5/08, A61K 9/00

(21) Application number: **97929947.6**

(22) Date of filing: **10.06.1997**

(86) International application number:
**PCT/US1997/010162**

(87) International publication number:
**WO 1997/047347 (18.12.1997 Gazette 1997/54)**

(54) **INHALER APPARATUS WITH MODIFIED SURFACES FOR ENHANCED RELEASE OF DRY POWDERS**

INHALATOR MIT MODIFIZIERTEN OBERFLÄCHEN ZUR VERBESSERTEN ABGABE VON TROCKENEN PULVERN

INHALATEUR AYANT DES SURFACES MODIFIEES POUR UNE MEILLEURE DISTRIBUTION DE POUDRES SECHES

(84) Designated Contracting States:
**AT BE CH DE DK ES FR GB IE IT LI LU NL PT SE**

(30) Priority: **10.06.1996 US 661213**
**10.06.1996 US 661212**

(43) Date of publication of application:
**26.05.1999 Bulletin 1999/21**

(73) Proprietor: **Delsys Pharmaceutical Corporation**
**Monmouth Junction, NJ 08552 (US)**

(72) Inventors:
 • **DATTA, Pabitra**
 **Cranbury, NJ 08512 (US)**
 • **RIVENBURG, Howard, Christopher**
 **Princeton, NJ 08540 (US)**
 • **DESAI, Nitin**
 **Princeton Junction, NJ 08550 (US)**

(74) Representative: **Pratt, Richard Wilson et al**
 **D Young & Co**
 **Briton House**
 **Briton Street**
 **Southampton SO14 3EB (GB)**

(56) References cited:
US-A- 4 072 129     US-A- 4 685 620
US-A- 4 795 644     US-A- 5 619 984

**Description**

In one aspect, the present invention provides an inhaler apparatus pursuant to claim 1 comprising interior surfaces having contact with a medicament for inhalation. According to an embodiment of the invention the interior surfaces include the interior of the mouthpiece and the substrate with medicament deposited thereon. According to the invention, at least one of such interior surfaces have indentations or raised areas therein, the raised areas having valleys between them. These surface modifications provide a mechanism for minimizing the area of contact between the medicament and the surfaces of the inhaler, thereby promoting release of the medicament from the inhaler.

Numerous approaches have been taken in the design and manufacture of dry powder inhalers. For example, WO 93/09832 discloses an inhalation device having an elongate carrier of medicament powder, the medicament powder being released after impact from a hammer, the inhalation device having a convoluted channel to deagglomerate the medicament powder.

The disadvantages of the inhalers of the prior art include, for example, the inability of a patient suffering from a respiratory disorder, such as asthma, to inhale with sufficient force to receive an entire dosage. For example, a patient may only be able to generate an air flow rate of about 15 liters per minute. In most dry powder inhalers, the patient's inhalation supplies the energy required to dispense the medicament from the inhaler. The air flow rate generated by the patient's lungs significantly affects the amount of medicament that ultimately exits the inhaler and reaches the lungs.

Another disadvantage of the inhalers of the prior art includes the inability to accurately determine the amount of medicament dispensed, since the inhaler may dispense a greater or lesser amount of medicament, depending upon the patient's air flow rate, for example.

A further disadvantage of the inhalers of the prior art is a problem of agglomeration of the medicament powder. Agglomerated particles generally impact the mouth and throat rather than remaining in the air flow for deposition on the lungs. One of the approaches to remedying this problem has been the provision of tortuous channels in the inhalers of the prior art to promote deagglomeration. This approach suffers from drawbacks, however, such as the deposition of the medicament along the channels, thereby leading to inaccurate dosage dispensing.

Another disadvantage encountered in the inhalers of the prior art is unintended dislodging, in which the medicament is discharged, for example, upon dropping the inhaler.

For the foregoing reasons, there is a need for a dry powder inhaler capable of delivering. an accurate unit dosage of medicament at a low flow rate, such as 15 liters per minute, yet which substantially retains the medicament upon impact, such as dropping the inhaler.

## SUMMARY OF THE INVENTION

The present invention is directed to an inhaler apparatus pursuant to claim 1. Preferred embodiments are specified in dependent claims. In certain preferred embodiments, the interior surface is a surface on a substrate having medicament deposited thereon, and in other preferred embodiments, the interior surface is also an interior surface of the mouthpiece of the inhaler. Preferably, the indentations or raised areas are of dimensions and spacings selected to reduce the amount of medicament particles that can contact a smooth surface of the substrate. Preferably, both the surface of the substrate and the mouthpiece and any other surfaces having contact with the medicament have indentations or raised areas therein, or any other surface structure for decreasing the area of contact between the selected medicament and the surface.

## BRIEF DESCRIPTION OF THE DRAWINGS

Figure 1 is a graphical representation of 3 forces that adhere particles to the substrate of the inhaler; electrostatic forces ("Fe"), charge imaging forces ("Fim") and van der Waals forces ("Fv").

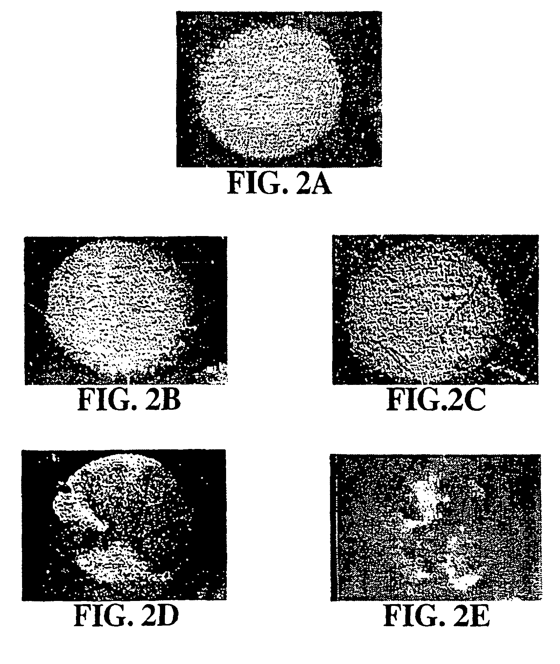

Figures 2A-E are micrographs of release of a powder from a polypropylene substrate with indentations therein, in the form of grooves. The magnification shown in Figures 2A-E is 42x. Figure 2A shows the powder before release; Figure 2B shows the powder remaining after being subjected to an air flow of 15 liters per minute; Figure 2C shows the powder remaining after being subjected to an air flow of 30 liters per minute; Figure 2D shows the powder remaining after being subjected to an air flow of 45 liters per minute; and Figure 2E shows the powder remaining after being subjected to an air flow of 57 liters per minute.

Figure 2F is a graphical representation of data obtained for the release of powder from the substrate shown in Figures 2A-E at increasing flow rates.

Figures 3A-C are photomicrographs of an inhaler substrate. Figure 3A is a photomicrograph of a polypropylene substrate with indentations therein, in the form of grooves; Figure 3B is a micrograph of the same substrate with powder deposited thereon, and Figure 3C is a micrograph of the same substrate after release of the powder.

Figures 4A-C are photomicrographs of an inhaler substrate made of silicon with grooved indentations in the surface as the substrate. Figure 4A is a photomicrograph of the substrate; Figure 4B is a micrograph of the same substrate with powder deposited thereon, and Figure 4C is a micrograph of the same substrate after release of the powder.

Figures 5A-C show a higher magnification of the photomicrographs of Figures 4A-C.

Figures 6A-C show a higher magnification of the photomicrographs of Figures 5A-C.

Figure 7 is a photograph of an embodiment of a mouthpiece of an inhaler of the invention, the arrow pointing to the air inlets of the mouthpiece.

Figures 8A and 8B are cross-sectional views of one embodiment of the inhaler apparatus of the invention. Figure 8A shows the inhaler without an electronic assisting means, and Figure 8B shows the inhaler with an electronic assisting means.

Figure 9 is a photograph of a set-up used to test release of the powder from the substrate of an inhaler.

Figure 10 is a graphical representation of the amount of medicament powder released from a planar substrate as compared to a substrate with grooved indentations therein.

Figures 11A and 11B are a diagrammatic cross-section of one embodiment of a substrate having medicament deposited thereon, the substrate being configured for electronically assisted release of the medicament.

Figures 12A and 12B are a diagrammatic cross-section of another embodiment of a substrate having medicament deposited thereon, the substrate being configured for electronically assisted release of the medicament.

Figure 13 is a cross-sectional schematic view of an electrostatic chuck with floating electrodes on the upper conductive layer for charge imaging.

Figure 14 is a top view of a floating electrode of Figure 13.

## DETAILED DESCRIPTION OF THE INVENTION

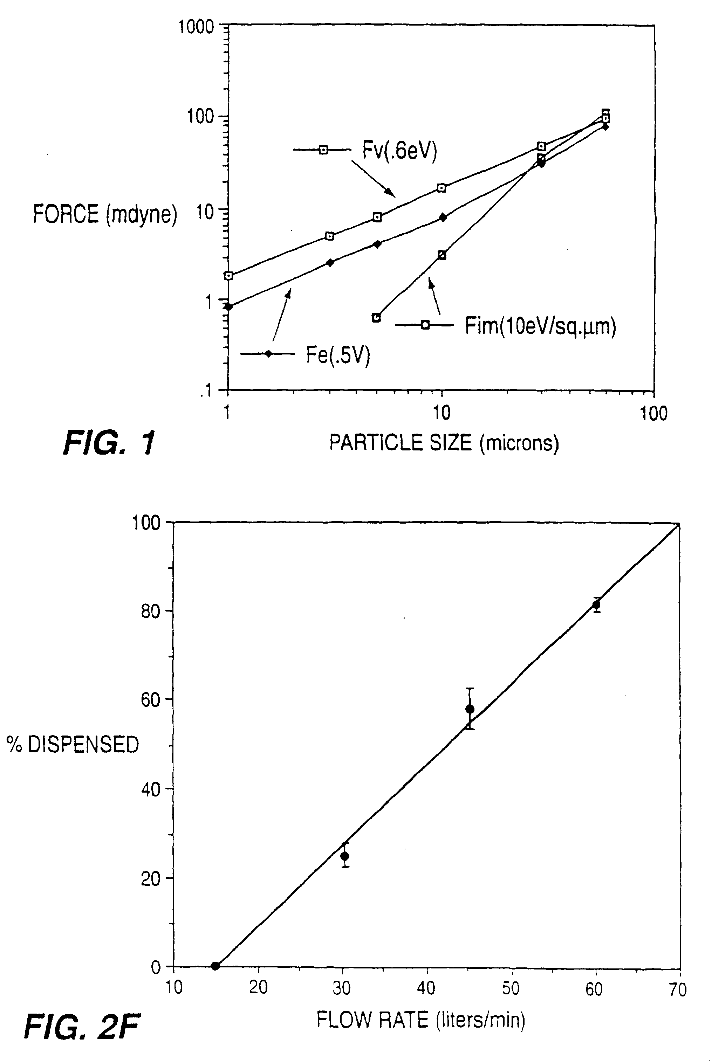

After depositing a powder onto a substrate of an inhaler, the powder is preferably accurately released upon inhalation by a patient. One of the obstacles to release that must be overcome is the adherence of the powder particles to the substrate. One of the forces holding the particles onto the substrate is a van der Waals force. Another one of the holding forces is the electrostatic force. A third holding force is a charge image force, generated by the charge of the powder particle in the local area of the substrate upon which it is adhered. It is believed that these forces vary in magnitude depending upon, for example, the conductivity of the substrate. It is believed that the van der Waals attraction increases over time, and the rate of increase is related to the rate of particle deformation due to greater contact area. Also, it is believed that these forces increase as the particle size increases. See, for example, Figure 1, which is a graphical representation of mathematical calculations of the foregoing forces.

In many embodiments of the present invention, the medicament is deposited on the substrate using an applied electric field to adhere charged medicament particles. After deposition, this applied field is preferably shut off and efforts are made to minimize the van der Waals force adhering the particles, thereby minimizing adhesive forces other than the image force.

### *Minimization of Attractive Forces*

The above-described problems are addressed, among others, by the current invention. In one aspect, the present invention provides for inhalers with modified substrates which alter the attractive forces. Preferably, greater than about 70%, and preferably greater than about 80% of the medicament is released upon inhalation. Preferably, the air flow required for release of about 80% to about 100% of the medicament in a dosage unit is less than about 60 liters per minute; more preferably, less than about 30 liters per minute, and even more preferably, no greater than about 15 liters per minute. See, for example, Figures 2A-E which show release of a medicament from a textured substrate having grooved indentations at 15 liters per minute (B), 30 liters per minute (C), 45 liters per minute (D), and 57 liters per minute (E). See also Figure 2F which is a graph of the data obtained and which shows the increasing release of medicament from the substrate as air flow increases. Example 1 provides the data used to generate the graph shown in Figure 2F. The deposition technique used in this example involved ion printing according to Serial No. 08/471,889. In preferred embodiments of the present invention, an electrostatic chuck is used to deposit electrostatically charged medicament onto the inhaler substrate, as described, for example, in U.S. Serial No. 08/630,050. A preferred deposition technique, using an electrostatic chuck, is believed to result in a higher percentage of release of the medicament from the inhaler substrate. Other deposition techniques can also be used with the modified inhaler substrates of the invention.

The inhaler substrate is preferably modified to minimize the surface area of the contact between the particles of the powder and the surface of the substrate, for those particles having a selected size. Particles having the desired size will have minimal contact with the substrate, and will therefore be more likely to be released from the substrate. In addition to making it more likely to release the desired particles, the modified substrate can be configured so that particles having an undesirable size are trapped. For example, if the surface area of contact between the particle and the substrate is high, such as with a particle having a size below the selected size, the higher contact leads to trapping the particle on the substrate rather than releasing it.

The minimization of the area of contact is preferably accomplished in the following ways. The surface area of contact can be minimized, for example, by providing indentations in the plane of the surface, or by providing raised areas in the plane of the surface. In preferred embodiments of the invention, at least one interior surface of the inhaler has indentations or raised areas with valleys there between, or other surface modification for decreasing the area of contact between the selected medicament particles and the interior surface of the inhaler in contact with the medicament. The contact of the medicament with the surface can occur, for example, before inhalation or during inhalation, such as contact with the substrate during deposition before inhalation, or contact with an interior surface of the mouthpiece during inhalation. Preferably, both the surface of the substrate upon which medicament is deposited and the mouthpiece and any other surfaces having contact with the medicament have indentations or raised areas therein, or any other surface structure for decreasing the area of contact between the selected medicament and the surface.

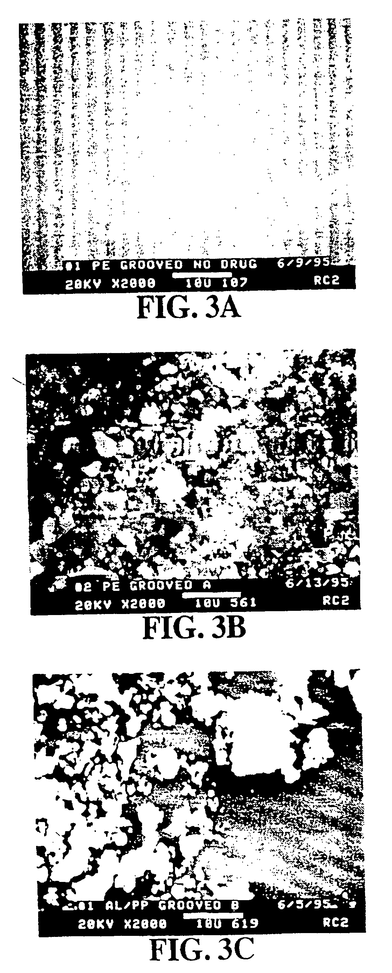

The indentation or raised area may be, for example, linear, tortuous, curved, circular, or any other desired configuration. In certain preferred embodiments, the indentations are in the form of linear grooves, which provides, for example, for ease of manufacturing. See, for example, Figures 3A-C, which show release from a polypropylene substrate having grooved indentations.

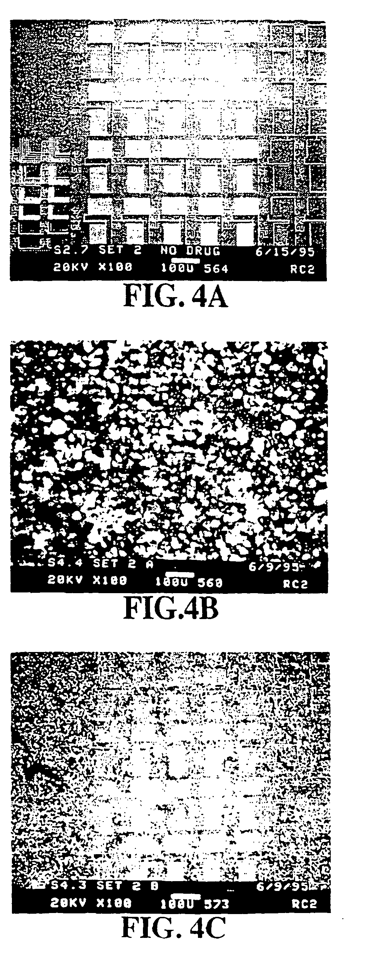

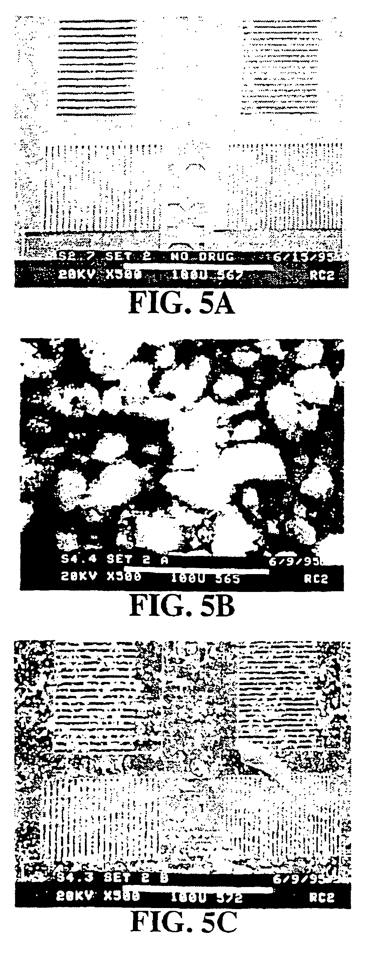

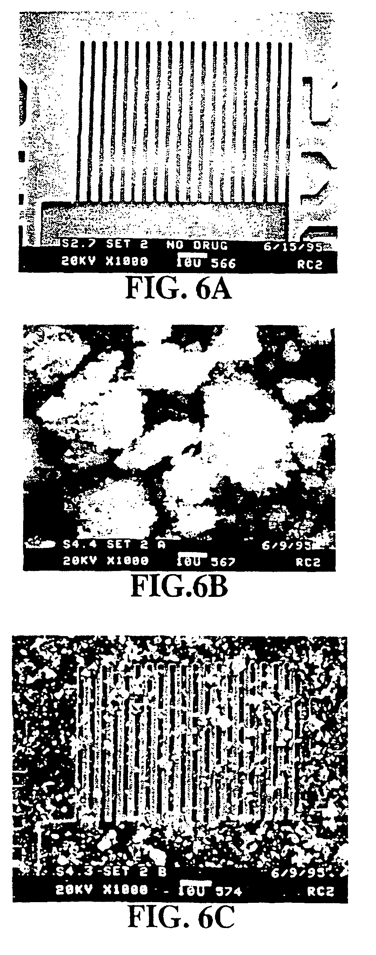

Specifically, Figure 3A is a micrograph of the substrate, which has grooved indentations therein, prior to deposition. Figure 3B is a micrograph of the substrate of Figure 3A after deposition of the medicament powder thereon. Figure 3C is a micrograph of the substrate of Figure 3B after release of the medicament from the substrate. Figures 4A-4C show the corresponding series of micrographs with a grooved silicon substrate. Figures 5A-5C and 6A-6C show the same series of materials, except at increasing magnifications. A 100 micron bar is provided in Figures 4A-4C and 5A-5C for size reference, and a 10 micron bar is provided in Figures 6A-6C.

Preferably, the depth of an indentation or the height of a raised area is slightly smaller than the size of the smallest particle desired to released from the inhaler, such as about 5% to about 50% smaller, and more preferably, about 5% to about 20% smaller than the smallest selected particle.

The width of the indentation or the valley between two raised areas is preferably slightly smaller than the diameter of the smallest particle selected to be released, such as about 5% to about 20% smaller, and more preferably, about 10% to about 20% smaller. For example, if the particles to be released from the inhaler have a selected size of about 2 to about 6 microns, the width of the indentation or valley will preferably be about 1.8 microns. Preferably, the diameter of the indentation or valley is less than the diameter of the minimum respirable medicament particle size. For example, the pitch of the substrate, measured from the center of a valley to the center of a raised area, is preferably about 1 to about 2.5 microns for dispensing particles from about 2 to about 6 microns. Particle size can be determined, for example, using scanning electron microscopy.

In addition to indentations and raised areas, the surface area of the contact between the medicament and the substrate may be decreased, for example, by using a perforated substrate. Furthermore, more than one such modification may be made to a single substrate. Preferably, the entire surface area of the surface in contact with the powder particles is modified to have minimized contact with the medicament powder.

A further aspect of the present invention is the use of a selected material to form the surface of the substrate in contact with the powder particles. Preferably, the material is selected in part on the basis of low surface energy. See, for example, Kaelble, *Physical Chemistry of Adhesion* at pages 149-164 (John Wiley & Sons 1971). Preferably, the surface energy of the surface in contact with the powder particles is between about 10 to about 25 dynes/cm. More preferably, the surfaces, when uncharged, have no substantial van der Waals or electrostatic interaction with the medicament. Furthermore, the material is preferably substantially chemically unreactive with the medicament. Examples of materials that can be used for such surfaces include perfluorinated polymers such as polytetrafluoroethylene ("TEFLON"), silicone, silicon alumina ceramic, polymeric photoconductor, polycarbonate, polyimide, polypropylene and polyethylene. In some embodiments, the surface has reacted with a silane, such as fluorosilane or aminosilane, to form a film having a low surface energy. Alternatively, for example, the surface can be treated to apply a perfluorinated polymer film. See, for example, U.S. Patent No. 4,252,848. See also, for example, the chapter entitled "The Properties of Fluorocarbon Films Prepared by Plasma Polymerization of 1,3-Perfluorodimethylcyclohexane" in S. Peprek and J. Hertz, eds., *4th International Symposium on Plasma Chemistry* (vol. 1 1979) at pages 152-163.

The material forming the surface in contact with the powder particles is also preferably selected on the basis of low chemical reactivity with the powder particles. For example, if the powder to be deposited upon the substrate is a charged or polar particle, the surface of the substrate is preferably not charged or polar. The materials used to form the surfaces in contact with the medicament are preferably selected to minimize the van der Waals and electrostatic adhesion of the medicament, as well as to minimize chemical reactivity.

Further, the material used to form the surface in contact with the medicament is preferably hard, and not pliable, particularly since pliability tends to increase contact area. See, for example, Nielsen, *Mechanical Properties*

*of Polymers and Composites* (Marcel Dekker Inc., NY 1974) at pages 367-369. Preferably, the material has a Vickers hardness greater than about 10 kp/mm$^2$, such as polystyrene, polymethyl methacrylate, polycarbonate, polyacetal, polyethylene terephthalate and phenolic resin.

Preferably, the material used to make a surface in contact with the medicament is a polymer. Preferred materials for use in such surfaces include polytetrafluoroethylene, silicon, alumina ceramic, aluminized organic photoconductor, polyvinyl carbazole, polycarbonate, polyimide and polyethylene. In certain embodiments, the indentations are the grooves present in an alumina ceramic printed board. See, for example, Figures 4-6. Such a printed board can be produced using standard photolithography techniques. In one embodiment, a die stamp having 2 micron spaced grooves is used to emboss a substrate, thereby creating a substrate with the desired indentations therein. See, for example, Figure 3.

In certain preferred embodiments, the surface is treated with a silane, such as fluorosilane or aminosilane. In some embodiments, polyimide is not preferred since in some instances, it may adhere a powder due to a chemical or electrostatic interaction. Preferably, the materials used and the surface treatment, if any, are pharmaceutically acceptable and do not cause substantial toxicity.

The size and shape of the substrate can be selected based upon the application. In some instances, for example, the substrate will be in the form of a disk or elongated such as a tape. Preferably, multiple dosage units are deposited onto the substrate, each dosage unit being in a discrete area, separated by an area of the substrate having no powder deposited thereon. In preferred embodiments, the substrate is sealed for protection, such as against the environment, including humidity, as well as for sterility.

The advantages of the inhaler apparatus of the present invention include its operation in releasing powder without the use of mechanical force, such as a hammer. The requirement of mechanical force to release the powder may mean that the powder is unintentionally released, for example, upon dropping the inhaler.

Although the inhalers of the present invention are designed for release of the medicament powder upon inhalation, preferably they do not release the medicament prior to inhalation. Preferably, for example, the medicament will remain on the substrate after the inhaler apparatus is subjected to a drop test, such as dropping the inhaler into a tube from a height of about 48 inches at a temperature of about 65 degrees Celsius and a relative humidity of about 65%.

In preferred aspects of the present invention, the inhaler apparatus further includes a mouthpiece with a configuration that prevents adherence of the medicament powder. For example, the mouthpiece preferably has an interior surface that is selected to resist adhering the powder particles. For example, the interior surface preferably has indentations or raised areas thereon, such as the modifications described above, to promote release of the powder. Preferably, the surface area of the interior surface of the mouthpiece is increased by using indentations in the form of grooves that are parallel to the direction of air flow in the mouthpiece, preferably causing substantially laminar air flow.

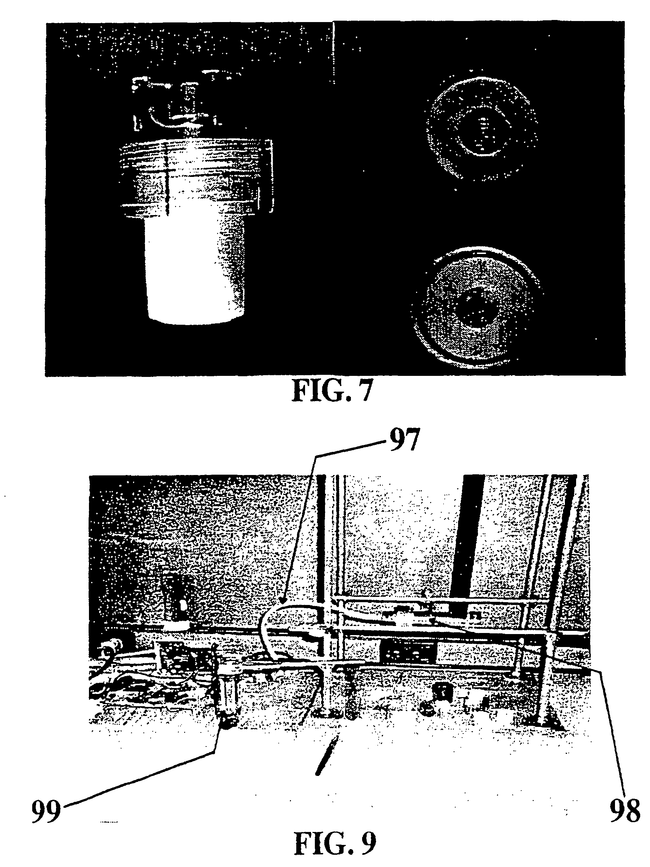

In additional preferred aspects of the present invention, the mouthpiece has multiple air inlets with a channel connected to each inlet for the enhancement of release of medicament powder. See, for example, Figure 7, in which the arrows point to the inlets. The channel connects the interior of the mouthpiece to the ambient atmosphere through an opening termed an "air inlet hole." Preferably, the air inlet hole is created, such as drilled, at an angle, preferably about 20 to about 70 degrees, and more preferably, about 45 degrees. Preferably, each channel extends from the corresponding air inlet at an angle of about 20 degrees to about 70 degrees. More preferably, the channel forms an angle of about 45 degrees from the horizon. In preferred embodiments, the channels are cylindrical and have a diameter of less than about 5 mm, such as about 0.1 to about 5 mm, or in some embodiments less than about 0.1 mm. Preferably, the mouthpiece is configured to maximize air flow between the powder and the substrate so that the powder is readily released from the substrate upon inhalation. In certain preferred embodiments, there are about 2 to about 20 air inlets and corresponding channels, and in other preferred embodiments, there are about 4 to about 8 air inlets.

Preferably, the air inlets can be opened and closed at will by the patient, or automatically via a shuttering mechanism, to maintain a constant pressure drop regardless of the air flow.

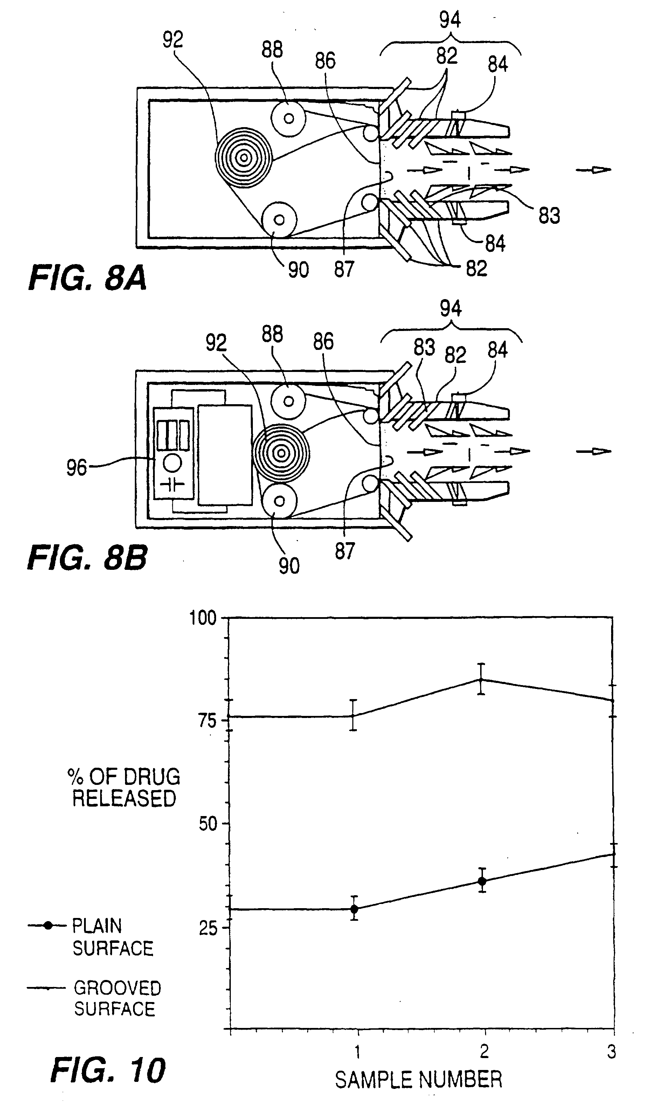

Illustrations of embodiments of the inhaler apparatus of the invention having multiple air inlets with channels connected to each inlet are provided in Figures 8A and 8B. Figure 8A shows a mouthpiece 94 with air inlets 82 having channels 83 attached thereto. A shuttering mechanism 84 is provided for several of the air inlets. The mouthpiece 94 is in air flow communication with the substrate 86 having medicant 87 (not shown) deposited thereon. The substrate 86 is in the form of an elongated tape, which is provided by reel 92 and taken up by reel 90. The substrate has a seal (not shown) which is taken up by reel 88. Figure 8B illustrates the inhaler of Figure 8A, further including an electronic release mechanism (not shown) powered by a battery 96.

In certain embodiments, each air inlet is connected via the channel to a portion of an individual dosage. For example, a dosage of 100 micrograms can be administered by aligning each of four 25-microgram dosages with each of four air inlets.

Preferably, only particles of the desired size, such as the respirable fraction, are deposited onto the substrate of the inhaler. Since the apparatus is preferably used with the medicament deposited in the desired particle range, and since in preferred embodiments, a substantial amount of undesired particle size range may be trapped on the substrate, there may be no need for additional devices to promote deagglomeration. Thus, the present invention provides advantages over inhalers requiring devices to deagglomerate, such as tortuous channels, that can trap medicament. In certain pharmaceutical applications, preferably the size of the particles dispensed by the inhaler is no greater than about 15 microns, and more preferably, no greater than about 10 microns.

In preferred embodiments, the substrate of the inhaler is equipped with a conductive layer for electronic assistance of release of the powder, as described in co-pending application entitled "Inhaler Apparatus with an Electronic Means for Enhanced Release of Dry Powders", filed simultaneously herewith.

The inhaler can also be equipped with other mechanisms for enhancing release, including an electron emitter such as a diamond tip emitter or other electron emitter, in order to neutralize the charge holding the powder onto the substrate. Alternatively, for example, the substrate upon which the medicament is deposited may be a photoconductive substrate that releases the medicament upon the application of light.

### Release Enhancement Using an Electric Field

The present invention is also directed, in part, to an inhaler apparatus comprising a substrate with a medicant deposited thereon, the substrate comprising a conductive layer and a dielectric layer thereon. In certain embodiments, the conductive layer comprises at least one wire embedded in the substrate, and in other embodiments, the conductive layer in the substrate has openings therein, such as a mesh. Preferably, the conductive layer is connected to a voltage source. Further, in certain preferred embodiments, the conductive layer comprises at least one floating electrode, and the medicant is preferably deposited on the substrate in a pattern determined by the floating electrodes.

In preferred embodiments, the inhaler further comprises a second conductive layer positioned above the substrate without having contact with the substrate. Preferably, the second conductive layer has openings therein, and more preferably, the openings of the second conductive layer have a diameter of about 1.5 times the average diameter of the particles of medicant. In certain preferred embodiments, the voltage source is connected to the conductive layer in the substrate and the second conductive layer above the substrate. Preferably, the openings in the conductive layer in the substrate, when present, have a diameter approximately equal to the diameter of the openings in the second conductive layer above the substrate. Preferably, the openings of the second conductive layer have a diameter of about 1.5 times the average diameter of the particles of medicant.

In other preferred embodiments, the inhaler apparatus further comprises a third conductive layer positioned below the substrate, and the voltage source is preferably connected to the second conductive layer above the substrate and the third conductive layer below the substrate.

In another aspect, the present invention provides a method for dispensing a medicant from an inhaler, comprising: (a) providing an inhaler with a substrate having a medicant deposited thereon, said substrate comprising a conductive layer and a dielectric layer and a voltage source connected to said conductive layer; and (b) actuating the voltage source.

Preferably, the voltage source is actuated substantially simultaneously with air flow, and the actuation is preferably a pulse having a duration of about 300 microseconds to about 1 millisecond. The voltage used is preferably from about 500 to about 2000 volts.

Thus, in another aspect, the present invention provides for inhalers with electronic means for enhanced release of dry powders. The electronic means for enhancing release is provided in preferred embodiments of the invention by a substrate of the inhaler comprising a conductive layer and a dielectric layer, the dielectric layer having contact with the powder deposited thereon. The conductive layer serves in one aspect to provide an image charge in response to the charge of the deposited medicament, thereby providing a force favoring the adherence of the medicament to the dielectric layer. Preferably, the dielectric layer is sufficiently thick to sufficiently shield the image force prevent the substrate from adhering the powder too tightly, but is also thin enough to allow a sufficient image force to prevent the powder from releasing prematurely, such as due to the force of impact if the inhaler is dropped.

For example, in order for a powder particle having a charge:mass ratio of q/m on a dielectric layer having a thickness d and dielectric constant $e_r$ to withstand a force of 500x gravity, $e_o$ being the dielectric constant of free space, and ignoring the van der Waals attraction, the following equation applies:

$$500x\ g \leq \frac{(q/m)^2 m}{4 p e_o e_r d^2}$$

Assuming, for example, that q/m = 30 mC/g, m = 7 pg and $e_r$ = 2, d can be at least as large as 76 mm. In reality, it is believed that in many cases the holding force will be stronger than 500x g due to the van der Waals attraction. The above equation can be used as a general guideline in determining the preferred thickness of the dielectric layer of the substrate.

The substrate of the inhaler has powder deposited thereon which is released upon inhalation. One means of powder deposition is ion printing, such as the technique described in Serial No. 08/471,889. Preferably, however, the substrate is not pre-charged prior to deposition of the medicament powder to attract the powder to the substrate. Instead, an electrostatic chuck is preferably used to electrostatically attract charged powder for deposition. For example, in certain preferred embodiments, the substrate itself forms an electrostatic chuck. Specifically, the conductive layer of the substrate has the configuration of an electrostatic chuck with floating electrodes for charge imaging, described in co-pending patent application Serial No. 08/630,050 (entitled "Electrostatic Chucks," filed April 9, 1996). The powder can be deposited on the substrate using an acoustic dispenser described in co-pending patent application Serial No. 08/630,049 (entitled "Acoustic Dispenser," filed April 9, 1996).

Briefly, an electrostatic chuck for charge imaging comprises three layers, preferably with an optional fourth layer. The bottom layer is the lower conductive layer, which is also known as the backing electrode. The second layer, on top of the lower conductive layer, is a dielectric layer. The third layer is an upper conductive layer on top of the dielectric layer, and this upper conductive layer has two types of electrodes, floating electrodes and shielding electrodes. In preferred embodiments, the floating electrodes are electrically isolated from the other conductors, and there is a gap between the floating and shielding electrodes. The fourth layer, on top of the upper conductive layer, is a dielectric layer, which is preferably the layer having contact with the medicament powder, the thickness of this layer being the subject of the above mathematical formula. Preferably, this layer is made of polyimide or another material of high dielectric strength. Without being limited to a particular theory, it is believed that when a potential is applied across the shielding and backing electrodes, a charge redistribution occurs on the floating electrodes. This charge redistribution causes electrostatically charged objects to be attracted to the areas of the chuck corresponding to the floating electrodes, thus resulting in deposition in these areas. Preferably, there is a high fringing field in the gap between the floating and shielding electrodes, but this field is preferably not large enough to cause electrical discharge.

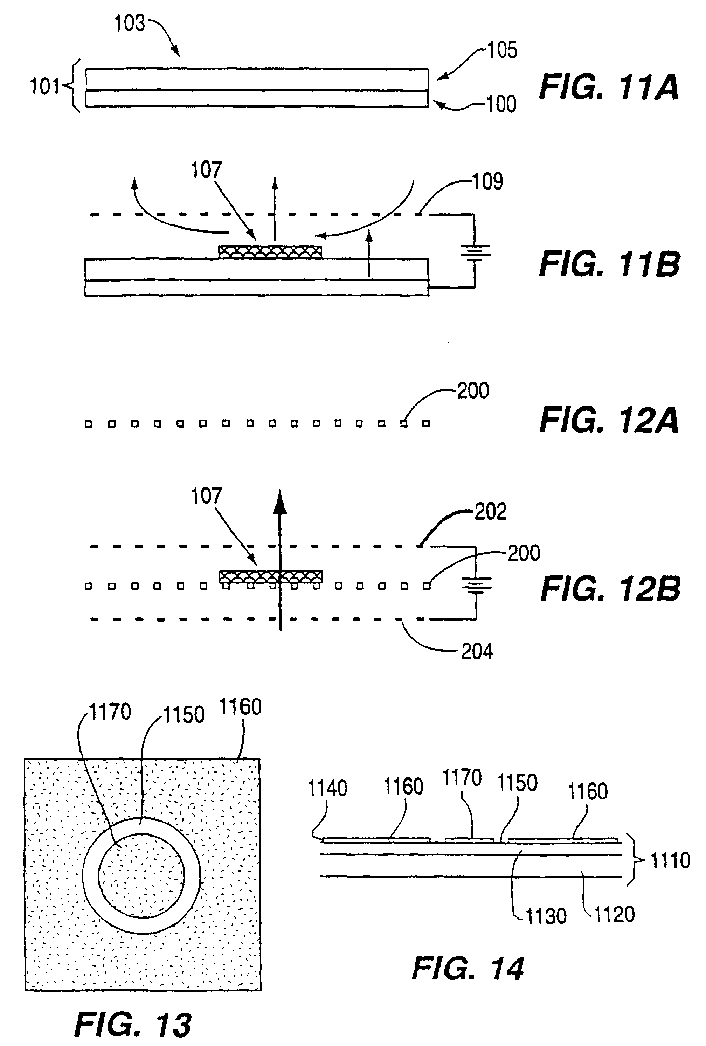

See, for example, Figure 13, which is a cross-sectional schematic view of an electrostatic chuck with floating electrodes on the upper conductive layer for charge imaging, Figure 14, which is a top view of a floating electrode of Figure 13. See also co-pending application U.S. Serial No. 08/630,050 "Electrostatic Chucks"), filed April 9, 1996. Referring to Figure 13, for example, the chuck 1110 has a lower conductive layer 1120, with a dielectric layer 1130 on top of it. The dielectric layer has an upper conductive layer 1140 on top of it. The upper conductive layer 1140 is electrically connected, but with a gap 1150 between a shielding electrode 1160 and a floating electrode 1170. A top view of the upper conductive layer 1140 is shown in Figure 14, with the floating electrode 1170 in the center, and a gap 1150 between the floating electrode and the surrounding shielding electrode 1160.

The floating electrodes of the charge imaging chuck determine the pattern of deposition of the medicament powder on the substrate, and hold the powder thereon. During the deposition of powder, the charge imaging chuck is electrically connected to a power source, which is subsequently disconnected after deposition. The floating electrodes can be configured, for example, to spatially determine individual dosages on the inhaler substrate. For example, the conductive layer 100 of the substrate 101 illustrated in Figures 11A and 11B can be a charge imaging chuck. The conductive layer of this chuck can also be used for the electronically assisted release of powder according to the present invention.

Specifically, Figures 11A and 11B are a diagrammatic cross-section of a substrate 101 having medicament deposited thereon, the substrate having a conductive layer 100 and a dielectric layer 105. The surface 103 of the substrate 101 in contact with medicament 107 is preferably modified according to co-pending application, filed simultaneously herewith, entitled "Inhaler Apparatus with Modified Surface for Enhanced Release of Dry Powders." During assisted release, a conductive layer 109, illustrated as a mesh, is positioned above the substrate and a voltage is applied across these two conductive layers, as shown in Figure 11B.

Thus, according to one aspect of the present invention, upon inhalation by a patient, the powder is released from the inhaler using an electronic mechanism to assist release. Preferably, the conductive material in the substrate is subjected to an electric pulse.

In certain preferred embodiments, such as when the substrate is solid, in order to release the medicament from the substrate of the inhaler, a potential is applied to the conductive layer in the chuck. Preferably, a conductive material, such as a mesh, is placed above the substrate during release, without having contact with the substrate, and an electric potential is applied between the substrate and the mesh. See, for example, Figure 11B.

A high voltage pulse is applied across the mesh and the conductive layer in the substrate to trigger release. Preferably, the pulse is synchronized with and triggered by air flow due to inhalation. For example, the inhaler preferably has a switch that activates the pulse upon air flow due to inhalation. The activation is preferably a pulse having a duration of about 300 microseconds to about 1 millisecond. The voltage used is preferably from about 500 to about 2000 volts.

The substrate can alternatively include a conductive layer that is not a charge imaging chuck. For example, the substrate may have a conductive layer with multiple holes, forming a mesh. For release, a second conductive layer having holes therein can be placed above and below the substrate, without having contact with the substrate. An electrical connection between the two conductive layers above and below the substrate provides for electronically assisted release of the powder from the substrate. See, for example, Figures 12A and 12B. Preferably, a conductive layer placed above or below the substrate is located from about 1 mm to about 2 mm from the substrate. During release, a high voltage pulse is applied across the two conductive meshes. Preferably, the pulse is synchronized with and triggered by air flow due to inhalation.

Figures 12A and 12B are a diagrammatic cross-section of a substrate 200 having medicament 107 deposited thereon, the substrate 200 being a mesh. During assisted release, two conductive layers 202 and 204, illustrated as meshes, with holes therein, are positioned above and below the substrate and a voltage is applied across these two conductive layers, as shown in Figure 12B. When the substrate has holes therein, two conductive layers, one above and one below the substrate, are preferably used to release the medicament from the substrate. Without being limited to a particular theory, it is believed that the use of the two conductive layers with a potential applied across them, enhances the release of powder from the substrates with holes therein in an upward direction, toward the mouthpiece. Preferably, the thickness of the substrate is about 1 mil to about 30 mils.

When the conductive material of the substrate has holes therein, such as a mesh, the holes are preferably configured to maximize air flow so that a substantial amount of the powder is released, and in preferred embodiments, are from about 600 microns to about 2 millimeters in diameter. Preferably, the mesh is made of a metal, such as stainless steel, and the mesh is preferably coated with a dielectric, such as polytetrafluoroethylene ("TEFLON"). The mesh can be a part of the mouthpiece, for example, and is preferably aligned with a single dose prior to inhalation.

Alternatively, for example, the substrate of the inhaler, upon which the medicament is located, can have embedded therein a solid conductive material. For example, the substrate may have embedded therein a wire or multiple wires. Preferably, the conductive material is a metal.

The power source for the electronic assistance is shown in the context of an exemplary inhaler in Figure 8B, which is a diagrammatic illustration of an embodiment of the inhaler apparatus of the invention having an electronic release mechanism (not shown) powered by a battery 96. In this embodiment, multiple air inlets 82 have channels 83 connected to each inlet to increase release of the powder from the mouthpiece 94. A shuttering mechanism 84 is provided for several of the air inlets 82. The mouthpiece 94 is in air flow communication with the substrate 86 having medicant deposited 87 thereon (not shown). The substrate 86 is in the form of an elongated tape, which is provided by reel 92 and taken up by reel 90. The substrate has a seal (not shown) which is taken up by reel 88.

The electronic assistance of release can be combined with other mechanisms for promoting release, including but not limited to the use of a substrate having grooved indentations to reduce the amount of surface area of the substrate in contact with the powder particles.

It will be understood by those skilled in the art that the inhalers of the invention can be used with numerous types of medicaments, and in addition to oral administration, the inhalers of the invention can be used with nasal administration. The biological agents that can be medicaments are generally substances such as chemicals that can act on a cell, virus, tissue, organ or organism, including but not limited to drugs (i.e. pharmaceuticals) to create a change in the functioning of the cell, virus, organ or organism. Preferably, the organism is a mammal, more preferably a human.

As mentioned above, the present application claims the priority of U.S. Application No. 08/661,212, filed June 10, 1996 (Attorney Docket DSRC 12212) and U.S. Application No. 08/661,212, filed June 10, 1996 (Attorney Docket DSRC 11894).

The present invention is further illustrated by the following non-limiting examples.

**Example 1. Release of Powdered Medicament from Modified Substrate**

A modified polypropylene substrate, as shown in Figure 3 was tested for release of a powdered medicament, mometasone furoate. A 2 cm$^2$ square of substrate was first weighed in milligrams on a microbalance ("sub(mg)"). Then, powdered medicament was deposited on the substrate, using the ion printing technique disclosed in U.S. Serial No. 08/471,889. The medicament was deposited in four dots, using several bursts of air to dispense a powder cloud. Next, the substrate was weighed with the medicament thereon ("sub+drug," which is provided in mg). The weight of the medicament ("drug(mg)") was determined by substracting the weight of the substrate before deposition ("sub(mg)") from the weight of the substrate after deposition ("sub+drug"). Two weight measurements were taken for each data point, and the two weight measurements were averaged ("average").

To dispense the powder, the substrate was placed in an apparatus such as that shown in Figure 9, and an inhaler mouthpiece was attached to the cylinder 98. The inhaler mouthpiece included 8 air inlets, each having a channel (capillary tubes) at a 45 degree angle from the mouthpiece to enhance lift off of the medicament powder. The release of the powder from the substrate was tested at four different flow rates of air applied to the substrate through the mouthpiece; 15, 30, 45 and 57 liters per minute. "Flow rate" indicates the air flow rate used to release the medicament from the inhaler. The substrate was weighed after release of the drug ("sub-drug," which is indicated in mg). The percentage of drug released from the substrate ("%drug") was determined using the weight of the drug left after release and the weight of the drug before release. "Humid./temp" indicates the percentage of humidity and ambient temperature (degrees Farenheit) at the time of the testing. The results are shown in Tables 1-2 below. Table 3 summarizes the data in Tables 1-2 by providing the average percentage of medicament release for each of the three flow rates, and the standard deviations. The data in Table 3 is depicted graphically in Figure 2F.

Table 1

| Sample | # appl/psi | sub+drug (mg) | sub (mg) substrate | drug(mg) (mg) | sub-drug after dispensing | Drug left (mg) | %drug dispensed | flow rate (liters/min) | humid./temp ./ |
|---|---|---|---|---|---|---|---|---|---|
| 1 | 5/7.5 | 8.7735 | 8.6095 | | 8.6494 | | | 57 | 68/81 |
| | | 8.7735 | 8.6092 | | 8.6495 | | | | |
| Ave. | | 8.7735 | 8.60935 | 0.16415 | 8.64945 | 0.0401 | 75.5711 | | |
| 2 | 5/7.5 | 7.2482 | 7.1406 | | | | | 57 | 67/81 |
| | | 7.2486 | 7.1409 | | | | | | |
| Ave. | | 7.2484 | 7.14075 | 0.10765 | 7.16 | 0.01925 | 82.1179 | | |
| | | | | | 8.2274 | | ( | | |
| 3 | 4/7.5 | 8.3174 | 8.1912 | | 8.2257 | | | n/d | 68/81 |
| | | 8.3161 | 8.1911 | | 8.2238 | | | | |
| Ave. | | 8.31675 | 8.19115 | 0.1256 | 8.225633333 | 0.03448 | 72.5451 | | |
| 4 | 4/7.5 | 6.8848 | 6.7813 | | | | | n/d | 68/81 |
| | | 6.8844 | 6.7816 | | | | | | |
| Ave. | | 6.8846 | 6.78145 | 0.10315 | n/d | | | | |
| 5 | 5/7.5 | 8.7858 | 8.681 | | | | | 45 | 68/81 |
| | | 8.7862 | 8.682 | | | | | | |
| Ave. | | 8.786 | 8.6815 | 0.1045 | 8.7215 | 0.04 | 61.7224 | | |
| 6 | 3/7.5 | 7.6297 | 7.5486 | | 7.5782 | | | n/d | 68/90 |

| | | | | | | | | | |
|---|---|---|---|---|---|---|---|---|---|
| | | 7.63 | 7.547 | | 7.582 | | | | |
| Ave. | | 7.62985 | 7.5478 | 0.08205 | 7.578133333 | 0.03033 | 83.0306 | | |
| 7 | 4/7.5 | 8.1118 | 7.9899 | | | | | 15 | 68/90 |
| | | 8.1117 | 7.9905 | | | | | | |
| Ave. | | 8.11175 | 7.9902 | 0.12155 | | | 6.3 | | |
| 8 | 5/7.5 | 7.517 | 7.4066 | | | | | 30 | 68/90 |
| | | 7.5168 | 7.4063 | | | | | | |
| Ave. | | 7.5169 | 7.40645 | 0.11045 | 7.4913 | 0.08485 | 23.1779 | | |

Table 2

| Sample | # appl/psi | sub (mg) (samp+subs) | sub+drug substrate | drug(mg) (mg) | sub-drug after dispensing | Drug left (mg) | %drug dispensed | flow rate (liters/min) | humid./temp temp (F)t |
|--------|-----------|---------------------|-------------------|---------------|---------------------------|----------------|-----------------|------------------------|------------------------|
| 9 | 6/7.5 | 7.0404 | 6.9084 | | 6.9337 | | | 60 | 67/83 |
| | | 7.0408 | 6.9086 | | 6.9344 | | | | |
| ave. | | 7.0406 | 6.9085 | 0.1321 | 6.93405 | 0.02555 | 80.658592 | | |
| | 4/7.5 | 7.5945 | 7.4758 | | 7.5067 | | | 45 | 66/83 |
| | | 7.5938 | 7.4765 | | 7.507 | | | | |
| ave. | | 7.59415 | 7.47615 | 0.118 | 7.50685 | 0.0307 | 73.983051 | | |
| 11 | 3/7.5 | 8.037 | 7.9513 | | 8.037 | | | 15 | 66/83 |

EP 0 917 476 B1

| Sample | # appl/psi | sub (mg) (samp+subs) | sub+drug substrate | drug(mg) (mg) | sub-drug after dispensing | Drug left (mg) | %drug dispensed | flow rate (liters/min) | humid./temp temp (F)t |
|---|---|---|---|---|---|---|---|---|---|
| | | 8.0371 | 7.9514 | | 8.0366 | | | | |
| ave. | | 8.03705 | 7.95135 | 0.0857 | 8.0368 | 0.08545 | 0.2917153 | | |
| 12 | 4/7.5 | 8.8213 | 8.7376 | | 8.8202 | | | 15 | 65/83 |
| | | 8.8207 | 8.7375 | | 8.8212 | | | | |
| ave. | | 8.821 | 8.73755 | 0.08345 | 8.8207 | 0.08315 | 0.3594967 | | |
| 13 | 5/7.5 | 7.1802 | 7.1081 | | 7.1794 | | | 15 | 65/83 |
| | | 7.1796 | 7.1081 | | 7.1795 | | | | |
| ave. | | 7.1799 | 7.1081 | 0.0718 | 7.17945 | 0.07135 | 0.6267409 | | |
| 14 | 3/7.5 | 6.8602 | 6.7494 | | 6.8293 | | | 30 | 70/84 |
| | | 6.8597 | 6.7485 | | 6.8294 | | | | |
| ave. | | 6.85995 | 6.74895 | 0.111 | 6.82935 | 0.0804 | 27.567568 | | |
| 15 | 5/7.5 | 9.3052 | 9.1824 | | 9.2381 | | | 45 | 70/86 |
| | | 9.305 | 9.1825 | | 9.238 | | | | |
| ave. | | 9.3051 | 9.18245 | 0.12265 | 9.23805 | 0.0556 | 54.667754 | | |
| 16 | 5/7.5 | 8.983 | 8.8723 | | 8.9582 | | | 30 | 71/86 |
| | | 8.9845 | 8.8727 | | 8.958 | | | | |
| ave. | | 8.98375 | 8.8725 | 0.11125 | 8.9581 | 0.0856 | 23.05618 | | |
| 17 | 7/7.5 | 9.3624 | 9.2077 | | 9.2326 | | | 60 | 70/86 |
| | | 9.3623 | 9.2081 | | 9.2324 | | | | |

EP 0 917 476 B1

| Sample | # appl/psi | sub (mg) (samp+subs) | sub+drug substrate | drug(mg) (mg) | sub-drug after dispensing | Drug left (mg) | %drug dispensed | flow rate (liters/min) | humid./temp temp (F)t |
|---|---|---|---|---|---|---|---|---|---|
| ave. | | 9.36235 | 9.2079 | 0.15445 | 9.2325 | 0.0246 | 84.072515 | | |

Table 3

| flow rate | 57 | 45 | 30 | 15 |
|---|---|---|---|---|
| | 80.658592 | 54.6677538 | 27.5675676 | 0.29171529 |
| | 84.0725154 | 73.9830508 | 23.0561798 | 0.3594967 |
| | 82.1179749 | 61.722488 | 23.1779086 | 0.62674095 |
| | | | | |
| ave. | 82.28302743 | 63.4577642 | 242.600552 | 0.425984313 |
| Standard deviation | 1.712936076 | 9.773871401 | 2.570231634 | 0.177132719 |
| | | | | |
| | 80.658592 | 54.6677538 | 27.5675676 | 0.29171529 |
| w/ one data point dropped | 84.0725154 | | 23.0561798 | 0.3594967 |
| | 82.1179749 | 61.722488 | 23.1779086 | 0.62674095 |
| | | | | |
| ave. | 82.28302743 | 58.1951209 | 24.600552 | 0.425984313 |
| standard deviation | 1.712936076 | 4.988450392 | 2.570231634 | 0.177132719 |

**Example 2. Comparison of Modified Substrate to Unmodified Substrate**

Approximately 50 mg dots of inhalation medicament were deposited on a 2 cm$^2$ polypropylene substrate using the ion printing process described in Serial No. 08/471,889 ("Methods and Apparatus for Electrostatically Depositing a Medicament Powder Upon Predefined Regions of a Substrate," filed June 6, 1995). The weight of the medicament was verified using a microbalance.

The release of medicament from an inhaler substrate having medicament deposited thereon was tested using the apparatus shown in Figure 9. Referring to Figure 9, air flow was generated through the use of a vacuum (not shown) attached to tubing 97, which was in turn attached to a cylinder 98, for attachment to an inhaler mouthpiece (not shown). The mouthpiece including 8 air inlets, each having a channel (capillary tubes) at 45 degree angles to the mouthpiece. A flow meter 99 was used to measure the rate of air flow. Three samples of each of two different substrates were tested, the first substrate having a grooved surface, as shown in Figure 3, and the second substrate having an unmodified planar surface. Both substrates were made of polypropylene. The results of the testing are shown in Table 4 below.

Table 4

| Sample number | % medicament released from grooved substrate | % medicament released from planar substrate |
|---|---|---|
| 1 | 80.5 | 62 |
| 2 | 84 | 64.5 |
| 3 | 82 | 67 |
| Ave. Value | 82.16 | 64.5 |
| Standard Deviation | 1.84 | 2.5 |

The data shown above is depicted graphically in Figure 10, which shows that release of the medicament from the substrate with indentations in the form of grooves was much higher than the release from an unmodified substrate.

**Claims**

1.    An inhaler apparatus having a mouthpiece **characterized in that** the inhaler apparatus includes an interior surface having contact with medicament particles (87) of a selected size range for inhalation, the interior surface comprising a substrate (86) including medicament particles of the selected size range deposited thereon, said interior surface being textured and comprising indentations or raised areas defining valleys of dimensions and spacings selected to decrease the area of contact between medicament particles of the selected size range and the textured surface, thereby reducing the adherence of the medicament particles to the textured surface.

2. The inhaler apparatus of claim 1, **characterized in that** the mouthpiece (94) has also an interior surface which is textured and comprises indentations or raised areas defining valleys of dimensions and spacings selected to decrease the area of contact between medicament particles of the selected size range and the textured surface, thereby reducing the adherence of the medicament particles to the textured surface.

3. The inhaler apparatus of claim 1, **characterized in that** the valleys have widths that are about 5% to about 20% smaller than a minimum selected particle size to be administered by the inhaler.

4. The inhaler apparatus of claim 1, **characterized in that** the valleys have a width of about 1 micron to about 2.5 microns.

5. The inhaler apparatus of claim 1, **characterized in that** the valleys have a depth that is about 5% to about 50% smaller than a minimum selected particle size to be administered by the inhaler.

6. The inhaler apparatus of claim 1, **characterized in that** the valleys are substantially regularly spaced throughout the area of the substrate having medicament particles thereon or throughout the mouthpiece (94) of the inhaler.

7. The inhaler apparatus of claim 1, **characterized in that** the valleys are substantially linear.

8. The inhaler apparatus of claim 1, **characterized in that** the substrate (86) comprises a disk or a tape.

9. The inhaler apparatus of claim 1, **characterized in that** the substrate (86) comprises multiple dosage units of medicament particles.

10. The inhaler apparatus of claim 1, further comprising a seal for sealing the substrate with medicament particles thereon.

11. The inhaler apparatus of claim 1, **characterized in that** the textured surface is made of a material having a low surface energy.

12. The inhaler apparatus of claim 1, **characterized in that** the textured surface is made of a material that is substantially chemically unreactive with the medicament particles.

13. The inhaler apparatus of claim 11, **characterized in that** the textured surface is made of a material selected from the group consisting of polytetrafluoroethylene, silicon, alumina ceramic, aluminized organic photoconductor, polycarbonate, polyimide, polypropylene and polyethylene.

14. The inhaler apparatus of claim 1, **characterized in that** the textured surface has a layer of silane thereon.

15. The inhaler apparatus of claim 14, **characterized in that** the silane is fluorosilane or aminosilane.

16. The inhaler apparatus of claim 1, **characterized in that** substantially all interior surfaces of the inhaler apparatus that have deposited medicament particles or which contact medicament particles during use have textured surfaces.

17. The inhaler apparatus of claim 1, **characterized in that** the pitch of the valleys is from about 1 micron to about 2.5 microns.

18. The inhaler apparatus of claim 1 **characterized in that** the mouthpiece has a wall with an exterior surface, the mouthpiece further comprising multiple air inlets (82) extending from the exterior to the interior, the inlets each being in communication with a channel (83), each channel extending from the interior to the exterior of the mouthpiece and each channel being positioned at an angle of about 20 to about 70 degrees from the wall of the mouthpiece.

19. The inhaler apparatus of claim 18, **characterized in that** the channels are positioned at an angle of about 45 degrees from the wall of the mouthpiece.

20. The inhaler apparatus of claim 18, **characterized in that** the channels are substantially cylindrical in shape.

21. The inhaler apparatus of claim 18, **characterized in that** the channels are less than about 5 mm in diameter.

22. The inhaler apparatus of claim 18, **characterized in that** the valleys are substantially parallel to the direction of airflow in the mouthpiece during inhalation.

23. The inhaler apparatus of claim 18 further comprising a shuttering mechanism for selectively closing at least one of the air inlets to control a pressure differential between the exterior and interior of the inhaler apparatus.

**Patentansprüche**

1. Inhalationsvorrichtung mit einem Mundstück, **dadurch gekennzeichnet, dass** die Inhalationsvorrichtung eine innere Oberfläche in Kontakt mit Arzneimittelteilchen (87) eines ausgewählten Größenbereiches für Inhalationszwecke umfasst, wobei die innere Oberfläche ein Substrat (86) umfasst, auf der Arzneimittelteilchen des ausgewählten Größenbereiches abgeschieden sind, wobei die innere Oberfläche strukturiert ist und Vertiefungen oder erhabene Bereiche umfasst, die Täler von Abmessungen und Abständen definieren, die so gewählt sind, dass die Kontaktfläche zwischen den Arzneistoffteilchen des gewählten Größenbereiches und der strukturierten Oberfläche verringert wird, wodurch das Haften der Arzneistoffteilchen an der strukturierten Oberfläche vermindert wird.

2. Inhalationsvorrichtung nach Anspruch 1, **dadurch gekennzeichnet, dass** das Mundstück (94) ebenfalls eine innere Oberfläche aufweist, die strukturiert ist und Vertiefungen oder erhabene Bereiche umfasst, die Täler von Abmessungen und Abständen definieren, die so gewählt sind, dass die Kontaktfläche zwischen den Arzneistoffteilchen des gewählten Größenbereiches und der strukturierten Oberfläche verringert wird, wodurch das Haften der Arzneistoffteilchen an der strukturierten Oberfläche vermindert wird.

3. Inhalationsvorrichtung nach Anspruch 1, **dadurch gekennzeichnet, dass** die Täler eine Breite aufweisen, die etwa 5 bis etwa 20 % kleiner ist als eine minimale gewählte Teilchengröße, die durch die Inhalationsvorrichtung zu verabreichen ist.

4. Inhalationsvorrichtung nach Anspruch 1, **dadurch gekennzeichnet, dass** die Täler eine Breite von etwa 1 μm bis etwa 2,5 μm aufweisen.

5. Inhalationsvorrichtung nach Anspruch 1, **dadurch gekennzeichnet, dass** die Täler eine Tiefe aufweisen, die etwa 5 bis etwa 50 % kleiner als eine minimale gewählte Teilchengröße ist, die durch die Inhalationsvorrichtung zu verabreichen ist.

6. Inhalationsvorrichtung nach Anspruch 1, **dadurch gekennzeichnet, dass** die Täler über den gesamten Substratbereich mit daran befindlichen Arzneistoffteilchen oder über das gesamte Mundstück (94) der Inhalationsvorrichtung in einem im wesentlichen regelmäßigen Abstand voneinander angeordnet sind.

7. Inhalationsvorrichtung nach Anspruch 1, **dadurch gekennzeichnet, dass** die Täler im wesentlichen linear sind.

8. Inhalationsvorrichtung nach Anspruch 1, **dadurch gekennzeichnet, dass** das Substrat (86) eine Scheibe oder ein Band umfasst.

9. Inhalationsvorrichtung nach Anspruch 1, **dadurch gekennzeichnet, dass** das Substrat (86) Mehrfachdosierungseinheiten von Arzneistoffteilchen umfasst.

10. Inhalationsvorrichtung nach Anspruch 1, ferner umfassend eine Dichtung zum Abdichten des Substrats mit den daran befindlichen Arzneistoffteilchen.

11. Inhalationsvorrichtung nach Anspruch 1, **dadurch gekennzeichnet, dass** die strukturierte Oberfläche aus einem Material mit einer geringen Oberflächenenergie gefertigt ist.

**12.** Inhalationsvorrichtung nach Anspruch 1, **dadurch gekennzeichnet, dass** die strukturierte Oberfläche aus einem Material gefertigt ist, das im wesentlichen mit den Arzneistoffteilchen nicht chemisch reaktiv ist.

**13.** Inhalationsvorrichtung nach Anspruch 11, **dadurch gekennzeichnet, dass** die strukturierte Oberfläche aus einem Material gefertigt ist, das aus der Gruppe Polytetrafluorethylen, Silicon, Aluminiumoxid-Keramik, aluminisierte organische Photoleiter, Polycarbonat, Polyimid, Polypropylen und Polyethylen besteht.

**14.** Inhalationsvorrichtung nach Anspruch 1, **dadurch gekennzeichnet, dass** sich auf der strukturierten Oberfläche eine Schicht aus Silan befindet.

**15.** Inhalationsvorrichtung nach Anspruch 14, **dadurch gekennzeichnet, dass** es sich bei dem Silan um Fluorsilan oder Aminosilan handelt.

**16.** Inhalationsvorrichtung nach Anspruch 1, **dadurch gekennzeichnet, dass** im wesentlichen sämtliche inneren Oberflächen der Inhalationsvorrichtung, auf denen Arzneistoffteilchen abgeschieden sind oder die während der Anwendung in Kontakt mit Arzneistoffteilchen stehen, strukturierte Oberflächen aufweisen.

**17.** Inhalationsvorrichtung nach Anspruch 1, **dadurch gekennzeichnet, dass** der Abstand der Täler etwa 1 µm bis etwa 2,5 µm beträgt.

**18.** Inhalationsvorrichtung nach Anspruch 1, **dadurch gekennzeichnet, dass** das Mundstück eine Wand mit einer äußeren Oberfläche aufweist, wobei das Mundstück ferner mehrfache Lufteinlässe (82) umfasst, die sich von außen nach innen erstrecken, wobei die Einlässe jeweils in Verbindung mit einem Kanal (83) stehen, wobei sich jeder Kanal vom Innenraum zum Außenraum des Mundstückes erstreckt und jeder Kanal in einem Winkel von etwa 20 bis etwa 70° zur Wand des Mundstückes angeordnet ist.

**19.** Inhalationsvorrichtung nach Anspruch 18, **dadurch gekennzeichnet, dass** die Kanäle in einem Winkel von etwa 45° zur Wand des Mundstückes angeordnet sind.

**20.** Inhalationsvorrichtung nach Anspruch 18, **dadurch gekennzeichnet, dass** die Kanäle eine im wesentlichen zylindrische Gestalt aufweisen.

**21.** Inhalationsvorrichtung nach Anspruch 18, **dadurch gekennzeichnet, dass** die Kanäle einen Durchmesser von weniger als etwa 5 mm aufweisen.

**22.** Inhalationsvorrichtung nach Anspruch 18, **dadurch gekennzeichnet, dass** die Täler im wesentlichen parallel zur Richtung des Luftstroms im Mundstück während der Inhalation angeordnet sind.

**23.** Inhalationsvorrichtung nach Anspruch 18, ferner umfassend einen Schließmechanismus zum selektiven Verschließen von mindestens einem der Lufteinlässe, um die Druckdifferenz zwischen dem Außenraum und dem Innenraum der Inhalationsvorrichtung zu steuern.

**Revendications**

**1.** Inhalateur comportant une embouchure, **caractérisé en ce que** ledit inhalateur comprend une surface interne en contact avec des particules de médicament (87) d'une gamme de dimensions sélectionnée pour l'inhalation, ladite surface interne comportant un substrat (86) qui contient lesdites particules de médicament de la gamme de dimensions sélectionnée qui ont été déposées sur ledit substrat, ladite surface interne étant texturée et présentant des indentations ou des zones surélevées qui définissent des sillons dont les dimensions et l'espacement sont choisis afin de réduire la surface de contact entre lesdites particules de médicament de la gamme de dimensions sélectionnée et ladite surface texturée, réduisant de cette façon l'adhérence des particules de médicament à la surface texturée.

**2.** Inhalateur selon la revendication 1, **caractérisé en ce que** l'embouchure (94) comporte également une surface interne texturée et couverte d'indentations ou de zones surélevées qui définissent des sillons dont les dimensions et l'espacement sont choisis afin de réduire la surface de contact entre les particules de médicament de la gamme de dimensions sélectionnée et la surface texturée, réduisant de cette façon l'adhérence des particules de médicament à la surface texturée.

**3.** Inhalateur selon la revendication 1, **caractérisé en ce que** les sillons ont des largeurs d'environ 5% à 20% plus petites que la dimension minimale sélectionnée pour les particules qui doivent être administrées par l'inhalateur.

**4.** Inhalateur selon la revendication 1, **caractérisé en ce que** les sillons ont une largeur d'environ 1 à 2,5 microns.

**5.** Inhalateur selon la revendication 1, **caractérisé en ce que** les sillons ont une profondeur d'environ 5% à 50% plus petite que la dimension minimale sélectionnée pour les particules qui doivent être administrées par l'inhalateur.

**6.** Inhalateur selon la revendication 1, **caractérisé en ce que** les sillons sont espacés de façon à peu près régulière sur la surface du substrat qui supporte les particules de médicament ou sur l'embouchure (94) dudit inhalateur.

**7.** Inhalateur selon la revendication 1, **caractérisé en ce que** les sillons sont sensiblement linéaires.

**8.** Inhalateur selon la revendication 1, **caractérisé en ce que** le substrat (86) comprend un disque ou une bande.

**9.** Inhalateur selon la revendication 1, **caractérisé en ce que** le substrat (86) comprend de multiples unités de dosage de particules de médicament.

**10.** Inhalateur selon la revendication 1, comprenant en plus un opercule pour sceller le substrat avec les particules de médicament.

**11.** Inhalateur selon la revendication 1, **caractérisé en ce que** la surface texturée est faite d'un matériau ayant une faible énergie de surface.

**12.** Inhalateur selon la revendication 1, **caractérisé en ce que** la surface texturée est faite d'un matériau chimiquement non réactif avec les particules de médicament.

**13.** Inhalateur selon la revendication 1, **caractérisé en ce que** la surface texturée est faite d'un des matériaux suivants : polytétrafluoroéthylène, silicium, céramique d'alumine, photoconducteur organique aluminisé, polycarbonate, polyimide, polypropylène et polyéthylène.

**14.** Inhalateur selon la revendication 1, **caractérisé en ce que** la surface texturée est recouverte d'une couche de silane.

**15.** Inhalateur selon la revendication 14, **caractérisé en ce que** le silane est du fluorosilane ou de l'aminosilane.

**16.** Inhalateur selon la revendication 1, **caractérisé en ce que** toutes les surfaces internes de l'inhalateur qui contiennent les particules de médicament ou qui sont en contact avec lesdites particules de médicament lors de l'utilisation ont des surfaces texturées.

**17.** Inhalateur selon la revendication 1, **caractérisé en ce que** l'espacement entre les sillons est d'environ 1 à 2,5 microns.

**18.** Inhalateur selon la revendication 1, **caractérisé en ce que** l'embouchure comporte une paroi avec une surface extérieure, ladite embouchure comprenant en plus de multiples prises d'air (82) qui s'étendent de l'extérieur vers l'intérieur, chacune desdites prises d'air étant en communication avec un canal (83), chacun desdits canaux s'étendant de l'intérieur vers l'extérieur de l'embouchure, et chacun desdits canaux formant un angle d'environ 20 à 70 degrés avec la paroi de l'embouchure.

**19.** Inhalateur selon la revendication 18, **caractérisé en ce que** les canaux forment en angle d'environ 45 degrés avec la paroi de l'embouchure.

**20.** Inhalateur selon la revendication 18, **caractérisé en ce que** lesdits canaux sont de forme cylindrique.

**21.** Inhalateur selon la revendication 18, **caractérisé en ce que** lesdits canaux ont un diamètre de moins de 5 mm environ.

**22.** Inhalateur selon la revendication 18, **caractérisé en ce que** les sillons sont parallèles à la direction du flux d'air dans l'embouchure lors de l'inhalation.

**23.** Inhalateur selon la revendication 18, comprenant en plus un mécanisme d'obturation afin de fermer de façon sélective au moins une des prises d'air pour contrôler la différence de pression entre l'extérieur et l'intérieur de l'inhalateur.

**FIG. 1**

**FIG. 2F**

FIG. 2A

FIG. 2B

FIG.2C

FIG. 2D

FIG. 2E

FIG. 3A

FIG. 3B

FIG. 3C

FIG. 4A

FIG.4B

FIG. 4C

FIG. 5A

FIG. 5B

FIG. 5C

FIG. 6A

FIG.6B

FIG. 6C

FIG. 7

FIG. 9

**FIG. 8A**

**FIG. 8B**

**FIG. 10**

SAMPLE NUMBER

% OF DRUG RELEASED

PLAIN SURFACE

GROOVED SURFACE

FIG. 11A

FIG. 11B

FIG. 12A

FIG. 12B

FIG. 13

FIG. 14